# EUROPÄISCHE PATENTSCHRIFT

(11) **EP 3 684 330 B1**
(45) Veröffentlichungstag und Bekanntmachung des Hinweises auf die Patenterteilung: **07.06.2023**
(21) Anmeldenummer: 18773440.5
(22) Anmeldetag: 20.09.2018
(51) Int. Cl.: A61K 8/42, A61Q 1/02, A61K 45/00, A61K 47/00, A61P 23/02

(54) **KOMBINATION VON TÄTOWIERTINTE UND EINEM ODER MEHREREN LOKALANÄSTHETIKA**
COMBINATION OF TATTOO INK AND ONE OR MORE LOCAL ANAESTHETICS
COMBINAISON D'ENCRE DE TATOUAGE ET D'UN OU DE PLUSIEURS ANESTHÉSIQUES LOCAUX

(30) Priorität: 21.09.2017 DE 102017121900
(43) Veröffentlichungstag der Anmeldung: 29.07.2020
(73) Patentinhaber: Toprak, Ferhan, 22041 Hamburg (DE)
(72) Erfinder: Toprak, Ferhan, 22041 Hamburg (DE)
(74) Vertreter: RGTH
(86) Internationale Anmeldenummer: PCT/EP2018/075474
(87) Internationale Veröffentlichungsnummer: WO 2019/057822

(56) Entgegenhaltungen:
- CN-U- 204 709 637
- DE-A1-102004 022 651
- DE-A1-102008 053 776
- US-A1- 2012 265 143
- Anonymous: "Infinitii Painless Tattoo Ink - 1oz Snow White", eBay , 18. September 2017 (2017-09-18), XP055516188, Gefunden im Internet: URL:https://www.ebay.com/itm/Infinitii-Pai nless-Tattoo-Ink-1oz-Snow-White/3114562616 09?hash=item48843d65e9:g:V1wAAOSwFnFWDGsi: rk:2:pf:0 [gefunden am 2018-10-17]
- Anonymous: "Mixing lidocaine into ink : tattoo", reddit , 30. März 2017 (2017-03-30), XP055515834, Gefunden im Internet: URL:https://www.reddit.com/r/tattoo/commen ts/62fv4z/mixing_lidocaine_into_ink/ [gefunden am 2018-10-16]
- DATABASE WPI Week 201606 Thomson Scientific, London, GB; AN 2015-703630 XP002785764, & CN 204 709 637 U 21. Oktober 2015 (2015-10-21)
- Anonymous: "Tattoo Anesthetic Options", Painfulpleasures Inc , 5. Juli 2015 (2015-07-05), XP055515830, Gefunden im Internet: URL:https://info.painfulpleasures.com/help -center/information-center/tattoo-anesthet ic-options [gefunden am 2018-10-16]
- Nico Krüger: "Lidocain - intravenöse Applikation zur perioperativen Analgesie", , 4 April 2017 (2017-04-04), XP55711792, Retrieved from the Internet: URL:https://siga-fsia.ch/files/Ausbildung/ Abschlussarbeiten/Hoehere_Fachschule_Zueri ch/2017/Diplomarbeit_Nico_Krueger_H15.pdf [retrieved on 2020-07-06]
- Anonymous: "Lokalanästhetika: im Orthopädischen Lexikon - Orthinform", , 12 January 2018 (2018-01-12), XP055711795, Retrieved from the Internet: URL:https://orthinform.de/lexikon/lokalana esthetika [retrieved on 2020-07-06]
- DATABASE MEDLINE [Online] US NATIONAL LIBRARY OF MEDICINE (NLM), BETHESDA, MD, US; July 2008 (2008-07), SURBATOVIC MAJA ET AL: "[Impact of hyaluronidase on anesthetic distribution in retrobulbar region following sub-Tenon anesthesia].", Database accession no. NLM18700462 & SURBATOVIC MAJA ET AL: "[Impact of hyaluronidase on anesthetic distribution in retrobulbar region following sub-Tenon anesthesia].", VOJNOSANITETSKI PREGLED JUL 2008, vol. 65, no. 7, July 2008 (2008-07), pages 525-531, ISSN: 0042-8450

## Beschreibung

Die vorliegende Erfindung bezieht sich auf eine Kombination von Tätowiertinte und einem oder mehreren Lokalanästhetika.

Eine Tätowierung ist ein Motiv, das mit Tinte, Farbpigmenten oder anderen Farbmitteln in die Haut eingebracht wird. Hierzu wird die Tätowiertinte, je nach dem gewünschten Effekt, durch eine oder mehrere Nadeln in die zweite Hautschicht gestochen. Das Motiv ist beliebig und kann ein einfarbiges oder mehrfarbiges Bild, Zeichen oder Symbol, Muster, Ornament oder auch Text darstellen. Die Tätowierung kann bei Mensch und Tier durchgeführt werden. Bei Haus- und Zuchttieren dient diese in der Regel zur Kennzeichnung und/oder Identifizierung und wird beispielsweise vom Tierarzt vorgenommen.

Beim Menschen werden Tätowierungen in der Regel von einem Tätowierer mit Hilfe einer elektrischen Tätowiermaschine vorgenommen. Tätowierungen gehören zu den sog. Körpermodifikationen ("Body Modifications" oder abgekürzt "BodMods"), die aus ästhetischen Gründen oder zur sexuellen Stimulation vorgenommen werden. Tätowierungen haben völlig unterschiedliche Funktionen und Bedeutungen für den Einzelnen oder auch eine Gruppe von Personen und können einen Ausdruck von Individualität oder Exklusivität repräsentieren. Tätowierungen können als Körperverzierungen und -verschönerungen, Permanent Make-up, als Schmuck, Zeichen der Zugehörigkeit zu einer Gruppe, wie z.B. bei Matrosen, Fremdenlegionären, Häftlingen, Yakuza oder Rockerbanden, als Protest, Statement oder Code, als modisches Accessoire, erotische Verzierung dienen und können auch als Kunstformen angesehen werden, wie Ganzkörpertätowierungen (sog. Bodysuits) und dergleichen. Auch im medizinischen Bereich können Tätowierungen eingesetzt werden, beispielweise zum Überdecken von Narbengewebe oder bei Brustrekonstruktionen.

In Deutschland ist etwa jeder Zehnte tätowiert, bei den 18- bis 29-Jährigen soll jeder Vierte ein Tattoo haben.

Anders als übliche Tattoos sind Henna-Tattoos oder temporäre Tattoos nur eine vorübergehende Körperbemalung, die nach einigen Wochen wieder verschwindet, wobei mit Henna-Farbe nur die oberste Hautschicht eingefärbt wird. Eine Tätowierung ist demgegenüber dauerhaft sichtbar, weil diese in die 2. Hautschicht oder Lederhaut gestochen wird. Während sich die darüber liegende oberste Hautschicht ständig erneuert, tut dies die Lederhaut nicht. Farbpigmente aus derTätowiertinte werden dort dauerhaft eingelagert.

Die Worte "Tätowieren" oder "Tattoo" haben ihren Ursprung in den polynesischen Sprachen. Das samoanische Wort "tatau" bedeutet "richtig" oder auch "gerade, kunstgerecht". Tätowierungen sind seit vielen tausend Jahren bei verschiedenen Völkern bekannt. So wurden bei der über 5000 Jahre alten Gletscher-Mumie Ötzi bereits Tätowierungen gefunden. Während früher das Tätowieren mit einfachsten Vorrichtungen, wie beispielweise meißelähnlichen Holzinstrumenten und spitzen Gegenstände, durchgeführt wurde, werden heutzutage modernste Techniken eingesetzt.

Zum Tätowieren benutzen Tattoo-Studios in der Regel eine elektrische Tätowiermaschine. Hiermit wird mit speziell geschliffenen Nadelspitzen die oberste Schicht oder Epidermis der Haut durchstochen und gleichzeitig die Tätowiertinte in die zweite Schicht oder Lederhaut, eingebracht. Der Einstich darf nicht nur oberflächlich und auch nicht zu tief sein. Wird das Färbemittel nur in die 1. Hautschicht oder Epidermis eingebracht, so würde aufgrund ständiger Erneuerung dieser Hautschicht ein Absondern der Farbteilchen gleichzeitig mit der Epidermis erfolgen. Wird zu tief gestochen, kommt es durch die auftretenden Blutungen zu einem Auswaschen der Färbemittel. Daher wird die Einstichtiefe in Abhängigkeit vom Hauttyp und der zu tätowierenden Körperstelle eingestellt.

Das Tätowieren stellt daher eine Punktierung der Haut dar, wobei gleichzeitig mit dem Durchstechen ein Färbemittel in die Haut eingebracht wird. In der Regel werden elektrische Tätowiermaschinen verwendet. Beispielsweise werden Spulenmaschinen verwendet, wobei mit bis zu 3 Spulen ein Magnetfeld erzeugt wird, durch das die Tätowiernadel vor und zurück bewegt wird. Dies erzeugt ein surrendes Geräusch, das den typischen Klang beim Tätowieren ausmacht. Eine weitere bekannte Tätowiermaschine arbeitet mit einem Elektromotor (Rotationstätowiermaschine oder Rotarymaschine, wie Cheyenne^{®} Hawk und dergleichen), der eine Auf- und Abwärtsbewegung erzeugt, ähnlich einer Stichsäge. Auch pneumatische Maschinen sind auf dem Markt erhältlich.

Die Tätowiermaschine sticht die in die Tätowiertinte getauchten Nadeln mit einer für das Auge nicht mehr sichtbaren Frequenz in die Haut. Die Geschwindigkeit ist abhängig von der Tätowiermaschine, der Technik und dem gewünschten Effekt, z.B. Linien, Schattierungen, Flächenanfärbung und dergleichen. Übliche Frequenzen liegen im Bereich von etwa 800 bis 7.500 Bewegungen pro Minute; in Einzelfällen können bis zu etwa 10.000 Stiche pro Minute in die Haut erfolgen. Linien und Umrisse können mit ein bis drei oder mehr Nadeln gestochen werden, bei Flächen können Blöcke mit bis zu 45 Nadeln zum Einsatz kommen.

Normalerweise sind Tattoo-Nadeln 0,3 bis 0,4 mm dick, es gibt jedoch auch Nadeln mit einem Durchmesser von 0,25 mm. Bekannt sind lange und kurze Nadeln, die mit verschiedenen Schliffen, sog. taper, angeboten werden. Bei den Nadeln gibt es beispielsweise "Liner" für Linien, "Shader" für Schattierungen und Magnum für große Flächen zum Schattieren oder Ausfüllen.

Beim Tätowieren kann zunächst eine Kontur des ausgewählten Motivs, beispielsweise mit schwarzer Farbe, vorgezeichnet werden, die dann mit den entsprechenden Farben und Flächen ausgefüllt wird. Die Wahl der verwendeten Nadeln, die Anzahl der Nadeln und deren Stärke sowie die verwendeten Tinten richten sich nach dem aufzubringenden Motiv und der angewandten Technik und werden entsprechend dem Können und der Erfahrung des Tätowierers verwendet. Die Grundtechnik des Tätowierens ist einfach: Die Haut wird mit einer Hand unter Spannung gehalten, die andere Hand führt die Nadel wie einen Stift und zeichnet. Allerdings ist die Haut strukturiert und nicht eben wie ein Blatt. Tätowieren erfordert daher Kunstfertigkeit, Geschick und viel Übung. Die Tinte hält sich dank einer Kapillarwirkung zwischen den Nadeln und wird durch die Schnelligkeit der Bewegung in die Haut gebracht. Anders als beim Injizieren werden beim Tätowieren keine Hohlnadeln verwendet.

Übliche Tätowiertinten umfassen ein oder mehrere Färbemittel, ein oder mehrere Trägerflüssigkeiten und gegebenenfalls Hilfsstoffe. Trägerstoffe sind Lösungs- und/oder Dispergiermittel, die beispielsweise aus destilliertem Wasser und Alkoholen ausgewählt werden können. In jüngster Zeit setzt sich jedoch destilliertes Wasser zunehmend durch.

Zur Erzeugung der Farbe sind beispielsweise Farbpigmente, wie Metallsalze, enthalten. Diese können wasserlöslich sein, sind aber häufig aufgrund ihres hydrophoben Charakters nicht wasserlöslich, so dass zusätzliche Lösungsvermittler, Lösungshilfsmittel und/oder Dispergiermittel eingesetzt werden, um die Pigmente in wässeriger Lösung zu dispergieren. Zum besseren Verständnis kann man angeben, dass beim Tätowieren etwa ein bis zwei Milligramm Farbpigment pro cm² Haut eingebracht werden.

Moderne Farben sind häufig frei von Alkoholen, basieren auf einer wässrigen Lösung, die Pigmente als Farbteilchen enthalten, und weisen eine gute Farb- bzw. Lichtechtheit auf. Die Farben sind insoweit gesundheitsverträglich, da diese auf Schwermetallbelastungen geprüft sind und keine krebserzeugenden aromatischen Amine aufweisen.

Als Tätowiertinten können handelsübliche Produkte verwendet werden, oder es können auch Mischungen aus handelsüblichen Produkten erzeugt werden.

Da die Tätowiertinte im Körper verbleibt, ist deren Qualität von besonderer Bedeutung. In Deutschland müssen Tätowiertinten seit 1. Mai 2009 die Tätowiermittel-Verordnung (TätoV) erfüllen, wonach bestimmte gesundheitsschädliche Inhaltsstoffe in den Farben/Pigmenten nicht mehr enthalten sein dürfen, wie beispielsweise krebserzeugende aromatische Amine.

Ein Problem beim Tätowieren ist, dass es sehr schmerzhaft ist, wenn Nadeln in die Haut eindringen. Der Schmerz ist eine natürliche Warnung des Körpers vor einer Verletzung, wobei jeder Mensch Schmerz anders empfindet und diesen auch unterschiedlich aushält. Ein leichtes Stechen kann für einen kein Problem sein, wohingegen ein anderer dies als unerträglich empfindet. Es liegt nur eine minimale Einstichtiefe bei gleichzeitig extrem kurzer Verweildauer der Nadel in der Hautoberfläche vor, so dass der Schmerz ähnlich zu demjenigen eines Nadelstichs beim Arzt bei einer Blutuntersuchung sein sollte. Jedoch wird der Schmerz häufig als deutlich unangenehmer empfunden.

Neben dem von Mensch zu Mensch unterschiedlichen Schmerzempfinden spielt auch die Körperstelle eine entscheidende Rolle, auf die ein Tattoo aufgebracht werden soll. Besonders empfindlich Stellen sind dort, wo zwischen Haut und Knochen wenig Gewebe, wie Fett oder Muskeln, ist und zugleich viele Nerven vorliegen. Empfindliche Bereich sind beispielsweise Bereiche um die Knie, entlang der Wirbelsäule, im Gesicht, am Kopf oder Hals. Weniger empfindlich sind dagegen die Oberschenkel, Oberarme oder Schultern, wo sich am häufigsten die Tattoos befinden.

Aus dem Stand der Technik sind zahlreiche Zusammensetzungen von Tätowiertinten in Kombination mit anderen Substanzen bekannt geworden:
So bezieht sich die US 2017/0 007 524 A1 auf Zusammensetzungen, umfassend eine personalisierende Substanz, wie ein biologisches Material, Sand, Erde, Metall, Wasser, Meerwasser, Weihwasser, synthetische oder biologische Polymere, Keramik, tierisches und pflanzliches Gewebe oder eine andere physiologische kompatible Komponente mit persönlicher Bedeutung für ein Individuum, sowie einen Träger zur Injektion in die Haut, wie Tattootinte. Als Zusatz wird u.a. Witch Hazel (Extrakt der Zaubernuss) beschrieben, wobei es sich um eine Heilpflanze handelt, die blutstillende, entzündungshemmende, adstringierende und Juckreiz stillende Wirkung hat. Eine schmerzstillende Wirkung von Witch Hazel ist nicht bekannt und wird auch nicht beschrieben.

Weiterhin beschreibt die DE 10 2004 022 651 A1 eine gebrauchsfertige Tattoo-Farbe auf der Basis von Alkohol und kosmetischen Pigmenten, wobei diese bei Bestrahlung von UV-, Schwarz- oder Blaulicht im Dunkeln leuchtet. Die beschriebenen Zusätze sind nur zur Förderung der Wundheilung und/oder wirken entzündungshemmend. Zur Entlastung von Schmerzen wird empfohlen, unter UV-, Schwarz- oder Blaulicht zu tätowieren (s. Absatz [0029]: Brückenabsatz S. 3/4). Schmerzstillende Mittel, insbesondere Lokalanästhetika, als Zusätze werden nicht beschrieben.

Die DE 10 2008 057 822 A1 betrifft eine Vorrichtung zur Aufbewahrung und dermalen Verabreichung einer Substanz. Da pharmazeutische Substanzen verabreicht werden können, werden u.a. auch Schmerzmittel (s. Absatz [0013]) im therapeutischen Bereich erwähnt. Auch der Einsatz der Vorrichtung zur Tätowierung wird für die Verwendung im kosmetischen Bereich beschrieben (s. Absatz [0014]). Eine gleichzeitige Verwendung von Tinte und Schmerzmittel, insbesondere einem Lokalanästhetikum, wird nicht beschrieben und ist auch aufgrund der vorgenommenen Unterscheidung zwischen therapeutischem Einsatz einerseits und kosmetischer Verwendung andererseits nicht beabsichtigt.

Ferner offenbart die DE 10 2008 053 776 A1 eine additive Pflegeformulierung für Tätowierfarben. Die Pflege der Haut hat nichts mit Betäuben der Haut zu tun, so dass dieses Dokument dem technischen Hintergrund angehört.

Aus dem Stand der Technik sind auch zahlreiche schmerzlindernde Zusammensetzungen bekannt:
So beschreibt die EP 1 863 468 B1 eine topische anästhetische Zusammensetzung zur topischen Anwendung zum Anästhesieren und Abdecken von Nervenenden bei einer offenen Wunde eines Patienten, wobei es sich bei der offenen Wunde um eine Lazeration, einen chirurgischen Einschnitt, eine Abschürfung, ein Geschwür oder eine Brandwunde handelt, umfassend: eine wirksame Menge eines ersten Lokalanästhetikums, das einen schnellen Wirkungseintritt aufweist, und eine wirksame Menge eines zweiten Lokalanästhetikums, das eine lange Wirkungsdauer aufweist, einen Vasokonstriktor in einer Menge, die zur Reduzierung einer Blutung aus der offenen Wunde und zur Verringerung der vaskulären Absorptionsrate der Lokalanästhetika wirksam ist, um das Risiko einer systemischen Toxizität zu reduzieren, ein Antiseptikum, einen detektierbaren Marker in Form eines Farbmittels, der das Vorhandensein von anästhetischen Wirkstoffen auf der offenen Wunde anzeigt, und einen mehrwertigen Alkohol in Kombination mit einem Cellulosepräparat als Geliermittel. Die Zusammensetzung liegt in Form eines sichtbar gefärbten, klebrigen, viskosen Sprühgels vor, das die offene Wunde abdecken kann und geeignet ist, eine Abgabe von Wirkstoffen in der Zusammensetzung an die offene Wunde dadurch zu maximieren, dass sie feucht und viskos bleibt, wenn eine solche offene Wunde damit abgedeckt wird, und wobei beim Auftragen des Gels auf Nervenenden in der Wunde eine Schmerzbetäubung unabhängig von den anästhetischen Wirkstoffen eintritt, wodurch es zur anästhetischen Wirkung der Zusammensetzung beiträgt und sie verstärkt.

Weiterhin bezieht sich die EP 2 620 144 A1 auf eine Färbezusammensetzung, umfassend einen vitalen Farbstoff und ein Anästhetikum. Die Zusammensetzungen werden zum flächigen Anfärben von Gewebe im Auge für eine Augenoperation eingesetzt. Es handelt sich daher um sehr speziell für das Auge geeignete Farbstoffe, die beispielsweise besonders gut an fibröses Gewebe, wie Gewebe der Netzhaut, binden können. Weiterhin ist die Färbung nur vorübergehend und verschwindet nach der Augenoperation wieder. Die Färbung wird daher zum Einfärben von ganzen Bereichen verwendet, um diese von umgebendem Gewebe bei einer Operation besser unterscheiden zu können.

Die US 2017/0143 618 A1 offenbart ein Verfahren zum Entfernen von Tattoos ohne Verwendung eines Lasers. Hierbei kommen auch Anästhetika zur Verwendung, wie beispielsweise durch Auftrag einer Creme (s. Anspruch 3) oder auch durch Auf-/Einbringen eines Anästhetikums in Form einer Flüssigkeit mit der/den Nadel(n) in die Haut (s. Absatz [0058]). Im Unterschied zur vorliegenden Erfindung wird das Verfahren erst nach dem Tätowieren eingesetzt und das Anästhetikum daher separat und unabhängig von der Tinte verwendet.

Ferner beschreibt die US 8 512 768 B2 eine schmerzlindernde Zusammensetzung, umfassend
0,5 - 7,0 Gew.-% Anästhetikum
1,0 - 11,0 Gew.-% entzündungshemmendes Mittel
0,5 - 12,0 Gew.-% feuchtigkeitspendendes Mittel
0,5 - 13,0 Gew.-% Feuchthaltemittel
0,5 - 7,0 Gew.-% Penetrations-verstärkendes Mittel und
40,0 - 97,0 Gew.-% Trägerlösungsmittel.

Die schmerzlindernde Zusammensetzung kann vor und/oder nach den folgenden Verfahren oder Behandlungen verwendet werden: Körperenthaarung mit Wachs, Rasieren, (Augenbrauen-)Zupfen, Laserhaarentfernung, epilierende Haarentfernung (Halawa), Tätowieren, Tattoo-Entfernung, Aufbringen von Permanent Make-up, Elektrolyse und Schmerzstillung bei Sonnenbrand. Somit kann die Zusammensetzung vor und/oder nach einem Tätowieren eingesetzt werden. Die Zusammensetzung wird auf die Haut aufgebracht, wo man diese einige Zeit einwirken lässt, wonach die Behandlung durchgeführt wird. Auch nach der Behandlung kann die schmerzstillende Zusammensetzung wieder auf die Haut aufgetragen werden. Demzufolge werden die Tätowiertinte und das schmerzlindernde Mittel hier nicht in einer Zusammensetzung zusammen verwendet, und die schmerzstillende Zusammensetzung wird auch nicht vor dem Tätowieren mit der Tätowiertinte zusammengebracht und dann gleichzeitig mit dieser verwendet. Die Zusammensetzung ist daher nicht speziell auf das Tätowieren abgestimmt.

Die Infinitii Painless Tattoo Ink - 1oz Snow White aus eBay vom 18. September 2017, (https://www.ebay.com/itm/Infinitii-Painless-Tattoo-Ink-1oz-Snow-White/3114562616 09?) beschreibt ein Produkt, das im Internet angeboten wurde und eine Zusammensetzung aus weißer Tätowiertinte und Lokalanästhetikum in Form von Lidocain darstellt. Es ist jedoch hieraus nicht ersichtlich, ob die Tätowiertinte und das Lokalanästhetikum tatsächlich eine gemeinsame Flüssigkeit bilden oder ob sich die Mischung nicht vielmehr in der Flasche des Produkts separiert. Das Produkt ist im Handel nicht mehr verfügbar.

"Mixing lidocaine into ink: tattoo" vom 30. März 2017 (https://www.reddit.com/r/ tattoo/comments/62fv4z/mixing_lidocaine_into_ink/) stammt aus einem Diskussions-Forum, wonach Lidocain in eine Tattotinte gemischt werden könnte. Es ist fraglich, ob dies eine ausführbare Lehre zum technischen Handeln darstellt.

Surbatovic Maja et al: in "[Impact of hyaluronidase on anesthetic distribution in retrobulbar region following sub-Tenon anesthesia]", Vojnosanitetski Pregled, Bd. 65, Nr. 7, Juli 2008, S. 525-531, offenbart in einer experimentellen Studie die Verwendung einer 4,5 ml-Mischung aus 2% Lignocain, 0,5% Bupivacain und 0,5 ml Indian Ink mit verschiedenen Mengen an Hyaluronidase für eine lokalanästhetische Betäubung bei Operationen des Auges durch Injektion in den Subtenon-Raum des Auges. Dies hat mit dem klassischen Tätowieren nichts zu tun. Die Tinte dient als eine Art Kontrastmittel, um die Verteilung des Lokalanästhetikums zu beobachten, wird im Anschluss wieder abgebaut und verschwindet daher. Die Tinte beim Tätowieren liegt demgegenüber permanent vor.

Die US 2012/265143 A1 beschreibt einen medizinischer Injektor zum Einspritzen von Füllsubstanzen in die Haut, d.h. es wird eine Hohlnadel zum Injizieren verwendet. Beim Tätowieren findet im Gegensatz hierzu keine Hohlnadel Verwendung. Weiterhin können gemäß Absatz [0056] das Medikament und/oder der dermale Füllstoff eine Tätowiertinte enthalten. Das Medikament und/oder der Füllstoff sind hierbei jedes Material, das das Weichgewebe vergrößert bzw. erweitert. Derartige Verbindungen sind beim Tätowieren nicht einsetzbar, da anschwellende Mittel die Haut deformieren würden, so dass kein Tattoo mehr aufgebracht werden könnte.

In der CN 204 709 637 U wird wieder eine Hohlnadel eingesetzt, die erfindungsgemäß nicht zum Einsatz kommt. Ferner wird ein "new ink storage space" beschrieben, wobei zusätzliche Tinte wohl über die Hohlnadel zugeführt wird. Es werden zwar "reduced epidermis narcotic phenomena" beschrieben, jedoch werden Lokalanästhetika nicht explizit erwähnt. Außerdem erfolgt die Kombination in der Nadel.

In 'Tattoo Anesthetic Options|Painfulpleasures Inc" vom 5. Juli 2015 (https://info.painfulpleasures.com/help-center/information-center/tattoo-anesthetic-options) wird eine anästhesierend wirkende Creme, ein Gel, Spray, Schaumspray oder auch eine Seife beschrieben, aber keine flüssige Zusammensetzung, die ein Lokalanästhetikum enthält.

Aus der Diplomarbeit von Nico Krüger: "Lidocain - intravenöse Applikation zur perioperativen Analgesie, 4. April 2017, (https://siga-fsia.ch/files/Ausbildung/ Abschlussarbeiten/Hoehere Fachschule Zuerich/2017/Diplomarbeit Nico Krueger H15.pdf und aus "Lokalanästhika: im Orthopädischen Lexikon - Orthinform", 12. Januar 2018 (https://orthinform.de/lexikon/lokalanaesthetika) geht hervor, das Lidocain nicht nur als Lokalanästhetikum, sondern auch als Antiarrhythmikum Verwendung findet und zudem eine opioidsparende, antithrombotische, muskelschwächende, antiallergische und entzündungshemmende Wirkung aufweist.

In der DE 10 2004 022651 A1 wird eine gebrauchsfertige Tattoo-Farbe auf Basis von Alkohol und kosmetischen Pigmenten beschrieben, die bei Bestrahlung mit UV-, Schwarz- oder Blaulicht im Dunkeln leuchtet. Die Tattoo-Farbe soll auf der Haut entzündungshemmend sein und die Wundheilung fördern. In Absatz [0021] sind pflanzliche Zusatzstoffe in der Leuchtfarbe zur Förderung der Wundheilung und/oder Entzündungshemmung angegeben, ohne diese im Einzelnen zu bezeichnen.

Die DE 10 2008 053 776 A1 offenbart eine Wirkstoffkombination, bestehend aus Wasser, Vitaminen, die in Pantothensäure umgewandelt werden, wie Dexpanthenol (Provitamin B5), und hautfeuchtigkeitsregulierenden Stoffen, wie Harnstoff. Die Wirkstoffkombination kann in einer Tätowierfarbe verwendet werden. Es soll eine wundheilungsfördernde Wirkung resultieren, die den Wundwasserfluss reguliert und die Schorfbildung reduziert. Es findet eine Art Folienbildung auf der Wunde statt, die den Juckreiz reduzieren soll. Dies ist während des Tätowierens unerwünscht, da eine Folienbildung auf der Haut das Aufbringen eines Tattoos beeinträchtigen kann.

Es besteht demnach ein Bedarf, das Tätowieren für Mensch und Tier zu verbessern und speziell eine oder mehrere Zusammensetzungen bereitzustellen, die insbesondere auf das Tätowieren abgestimmt sind und eine schmerzlindernde Wirkung zeigen.

Der Erfindung liegt die Aufgabe zugrunde, den Stand der Technik zu verbessern. Insbesondere soll das Tätowieren so durchgeführt werden können, dass die Schmerzen hierbei deutlich reduziert werden oder sogar ein völlig schmerzfreies Tätowieren durchgeführt werden kann.

Erfindungsgemäß wird diese Aufgabe gelöst durch Bereitstellen einer Kombination zum Tätowieren, umfassend eine Tätowiertinte und ein oder mehrere Lokalanästhetika für die Haut, wobei
die Tätowiertinte und die ein oder mehreren Lokalanästhetika zusammen in einer flüssigen Zusammensetzung vorliegen oder
die Tätowiertinte in einer ersten flüssigen Zusammensetzung und die ein oder mehreren Lokalanästhetika in einer zweiten flüssigen Zusammensetzung vorliegen, wobei ein Anteil der ersten Zusammensetzung und ein Anteil der zweiten Zusammensetzung vor und während dem Tätowieren zusammen in einer flüssigen Zusammensetzung vorliegen. Zudem umfasst die flüssige Zusammensetzung erfindungsgemäß noch ein entzündungshemmendes Mittel, das ausgewählt ist aus Vitamin F, Vitamin E, ungesättigten Fettsäuren, Rutin, Bioflavanoiden, Sanddorn-Öl, Olivenöl, Jojobaöl, ätherischem Kamillenöl, Betamethason, Dexamethason und Mischungen dieser.

Die Tätowiertinte und das oder die Lokalanästhetika werden in beiden Alternativen somit als Kombination, d.h. gemeinsam verwendet:
Das oder die Lokalanästhetika befinden sich daher gemäß der ersten Alternative zusammen mit der Tätowiertinte in einer einzigen flüssigen Zusammensetzung. Die Nadel(n) der Tätowiervorrichtung werden in die flüssige Zusammensetzung eingetaucht, und es wird mit dieser Zusammensetzung auf der oder den Nadeln das Tätowieren durchgeführt.

Gemäß der weiteren Alternative liegt die Tätowiertinte in einer ersten flüssigen Zusammensetzung vor, und das oder die Lokalanästhetika liegen in einer zweiten flüssigen Zusammensetzung vor, wobei die erste und zweite flüssige Zusammensetzung vor und während dem Tätowieren in Anteilen kombiniert werden. Hierzu werden die Nadel(n) der Tätowiervorrichtung in die erste und zweite flüssige Zusammensetzung jeweils nacheinander in dieser oder umgekehrter Reihenfolge eingetaucht, so dass beide Zusammensetzungen erst auf der Nadel oder den Nadeln der Tätowiervorrichtung zusammenkommen und dort in einer flüssigen Zusammensetzung zusammen vorliegen. Die auf der oder den Nadeln vorliegende flüssige Zusammensetzung wird dann zum Tätowieren verwendet, so dass wieder gleichzeitig Tätowiertinte und ein oder mehrere Lokalanästhetika verwendet werden.

Beide Alternativen haben unterschiedliche Vorteile:
So ist es einfacher, wenn nur eine einzige Zusammensetzung zum Tätowieren verwendet werden kann. Hierzu wird einfach eine übliche Tätowiertinte durch die erfindungsgemäße anästhesierende Tätowiertinte ausgetauscht. Die Abläufe beim Tätowieren müssen nicht geändert werden und es müssen keine zusätzlichen Hygienemaßnahmen getroffen werden.

Wenn zwei verschiedene flüssige Zusammensetzungen eingesetzt werden, wobei eine die Tätowiertinte und die andere das oder die Lokalanästhetika aufweist, hat dies den Vorteil einer größeren Variabilität. Beispielsweise kann die Dosierung des Lokalanästhetikums in einfacher Weise erhöht werden, falls die Konzentration sich beim Tätowieren als zu gering herausstellt. Hierzu wird die zweite flüssige Zusammensetzung einfach ausgetauscht und stattdessen eine höher konzentrierte Zusammensetzung verwendet. Zudem kann während des Tätowierens in einfacher Weise eine Änderung der Konzentration des oder der Lokalanästhetika zu höheren oder geringeren Konzentrationen erfolgen. Ein weiterer Vorteil ist, dass die separate anästhetisierende Lösung mit verschiedenen Tätowiertinten in unterschiedlichen Farbtönen kombiniert werden kann, so dass nicht bei jedem Farbwechsel eine neue anästhesierende Zusammensetzung verwendet werden muss. Hierzu wird während des Tätowierens einfach nur die Tätowiertinte ausgetauscht, aber die anästhesierende Lösung beibehalten.

Somit gelingt es erfindungsgemäß, die beim Tätowieren entstehenden Schmerzen bzw. die Schmerzempfindlichkeit der Haut während des Tätowierens deutlich zu reduzieren oder dieses nahezu schmerzfrei durchzuführen. Im Ergebnis wird der Tätowiervorgang hierdurch angenehmer, und es kann praktisch schmerzfrei tätowiert werden.

Von besonderem Vorteil bei beiden Varianten ist, dass die Kombination aus Tätowiertinte und Lokalanästhetikum derart ausgestaltet ist, dass diese auf den speziellen Vorgang des Tätowierens abgestimmt ist. So liegen die Tätowiertinte und das oder die Lokalanästhetika gleichzeitig vor, während tätowiert wird. Damit dringt das oder die Lokalanästhetika bis in die 2. Hautschicht vor, wo dieses seine anästhesierende Wirkung unmittelbar ausübt. Anders als bei anderen Lokalanästhetika, die nur lokal auf der Hautoberfläche wirken, wird die Schmerzlinderung in die Hautschichten hineingebracht, und wirkt so effektiver und speziell dort, wo der Schmerz beim Tätowieren entstehen kann.

Die zugesetzten entzündungshemmenden Mittel sind ausgewählt aus Vitamin F, Vitamin E, ungesättigten Fettsäuren, Rutin, Bioflavanoiden, Sanddorn-Öl, Olivenöl, Jojobaöl, ätherischem Kamillenöl, Betamethason, Dexamethason und Mischungen dieser.

Der Begriff "Tätowiertinte" wird im Rahmen der vorliegenden Erfindung synonym mit "Tätowierfarbe" oder "Tätowiermittel" verwendet und bedeutet eine gefärbte, gegebenenfalls intensiv gefärbte, Flüssigkeit zur Verwendung zum Tätowieren. Mit anderen Worten stellt dies die zum Tätowieren eingesetzte Zusammensetzung dar, um ein Motiv in die Haut zu tätowieren.

Die erfindungsgemäße Tätowiertinte enthält oder besteht aus ein oder mehreren physiologisch akzeptablen Färbemitteln, ein oder mehreren physiologisch akzeptablen Trägerstoffen sowie gegebenenfalls ein oder mehreren physiologisch akzeptablen Hilfsstoffen.

Eine physiologisch akzeptable Verbindung ist häufig aus dem Bereich der Pharmazie, Kosmetik oder der Lebensmitteltechnologie und angrenzenden Gebieten bekannt, beispielsweise in einschlägigen Arzneibüchern aufgelistet, wobei deren Eigenschaften einer physiologischen Anwendung nicht entgegenstehen. Insbesondere in Kontakt mit der Haut ist es zweckmäßig, wenn eine physiologisch akzeptable Verbindung keine unmittelbar nachteiligen und/oder schädlichen Einflüsse zeigt, insbesondere keine Reizungen, Allergien oder andere Probleme beim Verwender auslösen.

Die ein oder mehreren Färbemittel, die in der Tätowiertinte als farbgebende Verbindungen fungieren, sind erfindungsgemäß nicht besonders beschränkt, solange diese zur Tätowierung geeignet sind. Es kann jegliches Farbmittel zum Einsatz kommen, das für Mensch und Tier verträglich ist und keine besonderen gesundheitlichen Risiken darstellt.

Färbemittel, die in der erfindungsgemäßen Tätowiertinte eingesetzt werden können, sind öllöslich, öldispergierbar, wasserlöslich oder wasserdispergierbar. Geeignete Färbemittel sind beispielsweise organische oder anorganische Pigmente, natürliche oder synthetische Farbstoffe und Kombinationen hiervon.

Die Färbemittel können beispielsweise ausgewählt sein aus anorganischen Pigmenten, wie Metalloxiden, vorzugsweise Eisenoxiden, Titanoxiden, Aluminiumoxid, Zirkoniumoxiden, Kobaltoxiden, Ceroxiden, Nickeloxid, Nickel-Chrom-Oxiden, Zinkoxiden sowie zusammengesetzten Oxiden; oder Metallhydroxiden, wie Calciumhydroxid, Eisenhydroxiden, Aluminiumhydroxid, Chromhydroxid, Magnesiumhydroxid sowie zusammengesetzten Metallhydroxiden. Andere beispielhafte Färbemittel sind Preußischblau, Eisensulfide, Manganviolett, Carbon Black, Glimmer oder Kaolin. Bevorzugte anorganische Pigmente sind ausgewählt aus Eisenoxid rot, gelb oder schwarz, Titandioxid, Zinkoxid, Chromoxid/-hydroxid sowie Mischungen dieser. Um den gewünschten Endfarbton oder eine Farbschattierung zu erhalten, können die Tinten auch Mischungen mit unterschiedlichen Mengen verschiedener Pigmente enthalten. Beispielsweise können Pigmente in unterschiedlichen mineralischen Formen verwendet werden. Zum Beispiel kann Titandioxid in einer seiner mineralischen Formen, wie Anatas, Brookit oder Rutil oder Mischungen hiervon zum Einsatz kommen.

Die Färbemittel können auch organische, verlackte oder Lack-Pigmente ("lake pigments") sein, die durch Ausfällung eines natürlichen oder synthetischen Farbstoffs mit einem Metallsalz, wie Aluminium-, Calcium- oder Bariumsalz, erhalten werden. Diese sind zumeist öldispergierbar. Beispiele sind Lack-Pigmente auf Basis von Indigo- oder Karmin-Farbstoffen.

Die bekannten Pigmente weisen unterschiedliche Farben auf, so dass sich nahezu alle Farbtöne von weiß bis schwarz herstellen lassen. Es können bereits vorgemischte Farben verwendet werden, es können aber auch selbstgemachte Mischungen eingesetzt werden.

Gemäß einer bevorzugten Ausführungsform werden keine mikroverkapselten Färbemittel verwendet. Es sollen auch keine Metallteilchen in der Tätowiertinte enthalten sein.

Weitere Färbemittel, die eingesetzt werden können, sind die bekannten FD&C- und D&C-zertifizierten Farbstoffe.

Besonders bevorzugt sind Farbmittel, die bereits aus dem medizinischen Bereich, insbesondere Pharmabereich, oder Kosmetikbereich oder aus der Lebensmittelindustrie bekannt sind und in diesen Bereichen auch zum Einsatz kommen.

Als Trägerstoffe werden Lösungsmittel oder Lösungsmittelgemische eingesetzt, die völlig inert und physiologisch annehmbar sind. Beispielsweise sind diese ausgewählt aus Alkoholen, wie kurzkettige Alkohole, insbesondere Ethanol oder Glycerin, und Wasser. Das in der Tätowiertinte enthaltene Wasser ist in der Regel demineralisiertes oder destilliertes Wasser oder Wasser zu Injektionszwecken. Um die beim Tätowieren erforderlichen Hygienevorschriften zu gewährleisten, ist es zweckmäßig, sterile Lösungsmittel einzusetzen.

Bevorzugt ist die flüssige Zusammensetzung (Gesamt-Zusammensetzung), umfassend die Tätowiertinte und ein oder mehrere Lokalanästhetika, eine Zusammensetzung auf Wasser-Basis. Bevorzugt ist auch die erste und/oder zweite Zusammensetzung jeweils eine Zusammensetzung auf Wasser-Basis. Auf "Wasser-Basis" bedeutet, dass der Hauptteil des Trägerstoffs bzw. Lösungsmittels Wasser darstellt.

Der Begriff "Lösungsmittel" soll jede Art von flüssigem Trägerstoff umfassen, so dass eine Lösung resultiert. Eine "Lösung" soll nicht nur eine echte Lösung, sondern auch eine Dispersion umfassen, in der die Bestandteile in flüssiger Phase verteilt vorliegen.

Optional können in der Tätowiertinte Hilfsstoffe eingesetzt werden, welche je nach Art und verwendeter Menge die Eigenschaften der Tinte entsprechend variieren und in gewünschter Weise verändern. Beispielhafte Hilfsstoffe sind Lösungsvermittler oder Lösungshilfsmittel, Dispergiermittel, Bindemittel, Verdickungsmittel, Viskositätsregulatoren, Konservierungsmittel, Stabilisatoren, Emulgatoren, Antioxidationsmittel, Duftstoffe, Puffer, Mittel zur Regulierung des pH-Werts, Befeuchtungs- oder Feuchthaltemittel, Antiseptika und viele andere. Die entzündungshemmenden Mittel sind Hilfsstoffe, die aber nicht optional, sondern zwingend vorliegen.

Nachfolgend sind einige veranschaulichende Hilfsstoffe angegeben, ohne die Tätowiertinte hierauf zu beschränken:
- Lösungsvermittler oder Lösungshilfsmittel, Dispergiermittel, Emulgatoren oder Stabilisatoren, die beispielweise ausgewählt sind aus Castoröl, Cetylalkohol, Methylglucosesesquistearat (Emulsan II), Gummi arabicum, Lanolin, Propylenglykol- und Ethylenglykol-Derivaten, Sojalecithin, Fettsäureestern, fraktionierten Lecithinen, Tensiden, wie nichtionischen Tensiden, beispielsweise Blockpolymere aus Ethylen und Propylen (Poloxamer), Rizinusöl-Derivaten, Fettalkoholen, Fettsäuren und Mischungen dieser.
- Bindemittel, Verdickungsmittel und Viskositätsregulatoren, die beispielweise ausgewählt sind aus Carboxymethylcellulose, Hydroxyethylcellulose, Hydroxypropylmethylcellulose, Xanthan, Pektin, Polyvinylpyrrolidon, Povidon, Crospovidon und Schellack und Mischungen dieser.
- Befeuchtungs- oder Feuchthaltemittel, die beispielweise ausgewählt sind aus Glycerin, Sorbit, Pentaerythrit, Inosit, Xylit, Pyrrolidoncarbonsäure und deren Salze, Jojobaöl, Vitamin F, Panthenol und Mischungen dieser.
- Antioxidationsmittel, die beispielweise ausgewählt sind aus Vitamin E, Vitamin F, Vitamin C, Rutin, Resveratrol, Carnosolsäure, Chitosan, Flavonoiden, Gallaten, Anthocyanen, Carotinoiden, wie Vitamin A, oder L-Cystein und Mischungen dieser.
- Konservierungsmittel, die beispielweise ausgewählt sind aus Benzalkoniumchlorid, Benzoesäure, Benzethoniumchlorid, Benzylalkohol, Parabenen, Hydantoin, Kaliumsorbat, Phenoxyethanol, Natriumdehydroacetat, Natriumhydroxymethylglycinate, Natriummetabisulfit und Natriumsulfit.
- Mittel zur Regulierung des pH-Werts, wie Säuren oder Laugen, beispielsweise Natronlauge.
- Antiseptika, die beispielweise ausgewählt sind aus Captan, Chlorhexidin, Hexachlorophen, Triclosan, Triacetin und Mischungen derselben.

Selbstverständlich können auch andere als die oben aufgeführten Hilfsstoffe eingesetzt werden. Dem Fachmann sind die einzelnen Hilfsstoffe bekannt, so dass diese ohne weiteres in der geeigneten Menge ausgewählt und eingesetzt werden können.

Eine beispielhafte Tätowiertinte in Form der ersten flüssigen Zusammensetzung kann die folgende Zusammensetzung aufweisen:
30 bis 95 Gew% ein oder mehrere Lösungsmittel, bevorzugt Wasser;
1 bis 40 Gew% ein oder mehrere Färbemittel; und
0 bis 30 Gew% ein oder mehrere Hilfsstoffe.

Die Zusammensetzung der Tätowiertinte variiert in Abhängigkeit von der farblichen Zusammenstellung. Beispielsweise enthält schwarze Tätowiertinte in der Regel 8 bis 16 Gew.-% Farbpigment. Eine weiße Tätowierfarbe weist demgegenüber einen deutlich höheren Anteil an Farbpigmenten von etwa 35 Gew.-% auf.

Die Hilfsstoffe liegen bevorzugt im Bereich von 0 bis 30 Gew.-%, bevorzugter 0 bis 20 Gew.-%, insbesondere 0 bis 15 Gew.-%, in der Tätowiertinte vor. Größere Mengen sind in Einzelfällen ebenfalls möglich. Der Anteil an Hilfsstoffen, sowie die Art und Menge dieser hängt vom verwendeten Farbpigment, dessen Menge und dem gewünschten Farbton der Tätowiertinte ab. Eine schwarze Tinte weist daher meist geringere Anteile an Bindemitteln auf als eine weiße Tinte, wobei die weiße Tinte regelmäßig einen höheren Pigmentanteil als die schwarze Tinte hat.

Erfindungsgemäß sind entweder in der Tätowiertinten-Zusammensetzung an sich oder in einer separaten Zusammensetzung ein oder mehrere Lokalanästhetika vorhanden.

Lokalanästhetika sind Arzneistoffe, die als Schmerzmittel reversibel und örtlich begrenzt wirken. Die Lokalanästhesie ermöglicht als Alternative zur Allgemeinanästhesie oder Narkose die örtliche Schmerzausschaltung, die nur im gewünschten Umfang lokal erfolgt. Lokalanästhetika reduzieren die Erregbarkeit von sensiblen Nervenfasern, blockieren durch Interaktion mit einer Bindungsstelle an der Membraninnenseite des Natriumionenkanals den Einstrom von Natriumionen und verhindern damit die zur Erregungsleitung nötige Ausbildung von Aktionspotentialen. Es resultiert eine Blockierung der Reizweiterleitung der Nervenzellen. Dies bewirkt eine lokale Betäubung.

Zunächst kann jedes dem Fachmann bekannte Lokalanästhetikum oder eine Mischung dieser eingesetzt werden.

Besonders bevorzugte Lokalanästhetika sind ausgewählt aus der Gruppe, bestehend aus Lidocain, Mepivacain, Prilocain, Articain, Bupivacain, Dibucain, Ropivacain, Etidocain, Dyclonin, Procain, Benzocain, 2-Chlorprocain, Oxybuprocain, Tetracain, Fomocain, Pramocain, Kampfer, Lignocain, Etidocain, Levobupivacain, Oxyprocain, Hexylcain, Dibucain, Piperocaine, Butamben, Butambenpikrate, Dimethisoquinhydrochlorid, Diperodon, Dyclonin, Ketamin, p-Butylaminobenzoesäure, Pramoxin und deren pharmazeutisch akzeptable Salze sowie Mischungen dieser. Besonders bevorzugt sind Lidocain und Benzocain, deren pharmazeutisch akzeptable Salze, insbesondere deren Hydrochloride, sowie Mischungen dieser.

Ein beispielhafter Vertreter ist Lidocain (2-Diethylamino-N-(2,6-dimethylphenyl)-acetamid) aus der Gruppe der Amid-Lokalanästhetika, eines der am häufigsten eingesetzten Lokalanästhetika. Seine Wirkung setzt schnell ein und ist von mittellanger Dauer, z.B. etwa 1,5 bis 3h. Es gehört zu den Stoffen mit mittlerer lokalanästhetischer Potenz.

Die flüssige Zusammensetzung der Kombination kann hinsichtlich ihrer Wirkung im Hinblick auf Stärke der Wirkung, Einsetzen der Wirkung und Dauer der anästhetischen Wirkung variiert werden. Dies kann beispielsweise durch Auswahl der Konzentration und der Art der eingesetzten Lokalanästhetika erfolgen. Beispielsweise gibt es schnell wirkende und langsam wirkende Lokalanästhetika. Dies ist aus der pharmazeutischen Fachliteratur bekannt, so dass der Fachmann die geeigneten Lokalanästhetika ohne weiteres auswählen kann.

Das Lösungsmittel wird je nach Art und Konzentration des enthaltenen Lokalanästhetikums ausgewählt und ist bevorzugt Wasser.

Es kann jede geeignete Konzentration an Lokalanästhetikum in der flüssigen Gesamt-Zusammensetzung oder der zweiten flüssigen Zusammensetzung eingesetzt werden. Die Zusammensetzung kann bevorzugt 4 bis 20 Gew.-%, noch bevorzugter 6 bis 15 Gew.-%, ganz besonders bevorzugt 8 bis 12 Gew.-%, an einem oder mehreren Lokalanästhetika aufweisen. Je nach Schmerzempfindlichkeit des Einzelnen, zu tätowierender Körperstelle und Größe oder Komplexität des zu tätowierenden Motivs kann die Konzentration an Lokalanästhetikum beliebig eingestellt werden. Besonders bevorzugt liegen in der Gesamt-Zusammensetzung oder der 2. flüssigen Zusammensetzung niedrige Konzentrationen im Bereich von 4 bis 7,9 Gew.-% bei geringer Schmerzempfindlichkeit, mittlere Konzentrationen im Bereich von 8 bis 12 Gew.-% bei mittlerer Schmerzempfindlichkeit und hohe Konzentrationen im Bereich von 12,1 bis 20 Gew.-% bei hoher Schmerzempfindlichkeit vor.

Für das Lokalanästhetikum, wie z.B. Lidocain, wurde festgestellt, dass eine optimale Wirkung im Bereich um 10 Gew.-% erhalten wird. Eine mittlere Konzentration von beispielsweise 8 bis 12 Gew.-% findet daher ganz besonders bevorzugt Verwendung. Eine Verdoppelung der Konzentration auf einen Bereich von 20 Gew.-% bedeutet keine doppelt so starke Wirkung, aber dennoch eine deutlich stärkere Betäubung. Diese wird wahrscheinlich nur in Ausnahmefällen zum Einsatz kommen. Geringe Konzentrationen im Bereich von 4 Gew.-% oder mehr zeigen nur relativ geringe betäubende Wirkung. Es wird daher im Einzelfall zu entscheiden sein, welcher Konzentrationsbereich besonders geeignet ist.

Von Bedeutung ist in diesem Zusammenhang die Löslichkeit. So ist beispielweise Lidocain in Wasser nur schlecht löslich, so dass bevorzugt ein wasserlösliches pharmakologisch akzeptables Salz eingesetzt wird, wie das Hydrochlorid.

Die Erfindung bezieht sich daher auch auf eine lokalanästhesierend wirkende Tätowiertinte, umfassend ein oder mehrere physiologisch akzeptable Färbemittel, ein oder mehreren physiologisch akzeptable Trägerstoffe als Lösungsmittel, die beschriebenen entzündungshemmenden Mittel, gegebenenfalls ein oder mehrere physiologisch akzeptable Hilfsstoffe, und ein oder mehrere Lokalanästhetika in einer flüssigen Zusammensetzung (Gesamt-Zusammensetzung), die bevorzugt auf Wasser-Basis ist.

Die Erfindung bezieht sich auch auf eine Tätowiertinte als eine erste flüssige Zusammensetzung, umfassend ein oder mehrere physiologisch akzeptable Färbemittel, ein oder mehrere physiologisch akzeptable Trägerstoffe als Lösungsmittel, die beschriebenen entzündungshemmenden Mittel und gegebenenfalls ein oder mehrere physiologisch akzeptable Hilfsstoffe, in Kombination mit ein oder mehreren Lokalanästhetika in einer zweiten flüssigen Zusammensetzung, die bevorzugt auf Wasser-Basis ist.

Besonders vorteilhaft ist eine erfindungsgemäße Kombination, umfassend eine Tätowiertinte und ein oder mehrere Lokalanästhetika und die beschriebenen entzündungshemmenden Mittel in ein und derselben flüssigen Zusammensetzung, welche bevorzugt die folgenden Bestandteile aufweist:
30 bis 95 Gew% ein oder mehrere Lösungsmittel, bevorzugt Wasser;
1 bis 35 Gew% ein oder mehrere Färbemittel;
0 bis 15 Gew% ein oder mehrere Hilfsstoffe; und
4 bis 20 Gew% ein oder mehrere Lokalanästhetika.

Tätowiertinten sind häufig vom Volumen her etwa zur Hälfte aus Färbemittel und zur anderen Hälfte aus Lösungsmitteln aufgebaut. Häufig verwendete Hilfsstoffe sind Verdicker und Konservierungsmittel. Jede Tätowiertinte hat ihre Besonderheiten bei der Verwendung.

Zum Tätowieren kann nur eine Tätowiertinte zum Einsatz kommen, wie beispielsweise bei einem Text oder Wort. Selbstverständlich können auch mehrere Tätowiertinten in unterschiedlichen Farbtönen zum Einsatz kommen. Jede dieser Tätowiertinten kann in ihrer flüssigen Zusammensetzung ein oder mehrere Lokalanästhetika aufweisen oder es kann in Kombination mit einer jeden Tätowiertinte jeweils eine lokalanästhetische Lösung eingesetzt werden. Aus hygienischen Gründen kann es vorteilhaft sein, wenn für jede Tätowierung jeweils eine eigene lokalanästhetische Lösung separat bereitgestellt wird.

Gegenstand der Erfindung ist daher auch die Kombination der vorliegenden Erfindung, umfassend eine Tätowiertinte mit den beschriebenen entzündungshemmenden Mitteln und ein oder mehrere Lokalanästhetika für die Haut zusammen in einer flüssigen Zusammensetzung oder die Tätowiertinte mit den beschriebenen entzündungshemmenden Mitteln in einer ersten flüssigen Zusammensetzung und die ein oder mehreren Lokalanästhetika in einer zweiten flüssigen Zusammensetzung, zur Schmerzlinderung oder -vermeidung während dem Tätowieren.

Die vorliegende Erfindung bezieht sich daher auch auf die Kombination von Tätowiertinte und ein oder mehreren Lokalanästhetika zur Schmerzlinderung oder - vermeidung während dem Tätowieren,
wobei die Tätowiertinte mit den beschriebenen entzündungshemmenden Mitteln und den ein oder mehreren Lokalanästhetika zusammen in einer flüssigen Zusammensetzung eingesetzt werden und
vor und während dem Tätowieren ein oder mehrere Nadeln einer Tätowiervorrichtung in die flüssige Zusammensetzung eingetaucht werden;
   oder
die Tätowiertinte mit den beschriebenen entzündungshemmenden Mitteln in einer ersten flüssigen Zusammensetzung und die ein oder mehreren Lokalanästhetika in einer zweiten flüssigen Zusammensetzung eingesetzt werden und vor und während dem Tätowieren ein oder mehrere Nadeln einer Tätowiervorrichtung
zunächst in die erste und dann in die zweite flüssige Zusammensetzung oder
zunächst in die zweite und dann in die erste flüssige Zusammensetzung eingetaucht werden,
bevor der jeweilige Tätowiervorgang durchgeführt wird.

Die Verwendung von erster und zweiter flüssiger Zusammensetzung ist der Verwendung eines ,kit of parts' aus dem pharmazeutischen Bereich vergleichbar, wobei die Verwendung hier jedoch im kosmetischen Bereich erfolgt.

Die Reihenfolge, ob die Nadel oder Nadeln der Tätowiervorrichtung zunächst in die erste und dann in die zweite flüssige Zusammensetzung oder umgekehrt eingetaucht werden, ist beliebig. Aus Zweckmäßigkeit und hygienischen Überlegungen kann es vorteilhaft sein, wenn zunächst in die zweite Zusammensetzung und dann in die erste Zusammensetzung eingetaucht wird, um die Tätowiertinte nicht in die anästhesierend wirkende Zusammensetzung zu verschleppen, so dass diese erneut zusammen mit einer weiteren Tätowiertinte verwendet werden könnte.

Das Tätowieren ist kein kontinuierlicher Vorgang, sondern es wird immer wieder unterbrochen, um mit der/den Nadel/n Tinte aufzunehmen. Daher ist es zweckmäßig, wenn bei jeder Unterbrechung des Tätowierens die Nadel/n wieder in die flüssige Zusammensetzung, umfassend Tätowiertinte und ein oder mehrere Lokalanästhetika eingetaucht wird/werden. Wenn zwei flüssige Zusammensetzungen vorliegen, ist es zweckmäßig, wenn bei jeder Unterbrechung der Tätowierung die Nadel/n in beide Zusammensetzungen nacheinander eingetaucht wird/werden. Demnach wird das Eintauchen der ein oder mehreren Nadeln in eine flüssige Zusammensetzung oder beide flüssige Zusammensetzungen während des Tätowierens wiederholt, bevorzugt bei jeder Unterbrechung des Tätowierens, bis das Tätowieren beendet ist.

Gemäß einer bevorzugten Ausführungsform der Erfindung kann das Tätowieren wie folgt durchgeführt werden:
Bevor mit dem Tätowieren begonnen wird, werden das Motiv und die gewünschten Farben ausgesucht.

Vor dem Tätowieren werden die für das Motiv ausgewählten Tätowiertinten hergerichtet, entsprechend vorbereitet und gegebenenfalls angerührt. Entweder kann der Tätowierer die Tinten vor dem Tätowieren in kleine Einmalbehälter umfüllen, oder er verwendet Einmal-Farbtuben. Wird als Lösungsmittel Wasser eingesetzt, so sollte dieses steril sein.

Die ein oder mehreren zu verwendenden Tätowiertinten können übliche Tätowiertinten sein, die zusätzlich jeweils ein oder mehrere Anästhetika aufweisen. Dies kann beispielsweise Lidocain sein. Andere Lokalanästhetika oder Mischungen dieser können ebenfalls eingesetzt werden.

Überraschenderweise wird durch Zusatz eines oder mehrerer Lokalanästhetika keine Verschlechterung der Farb- und Lichtechtheit der Tätowiertinten beobachtet. Vielmehr bleibt die ursprüngliche Qualität erhalten.

Beispielsweise kann das oder die Lokalanästhetika in einer Konzentration im Bereich von 4 bis 20 Gew.-%, noch bevorzugter 6 bis 15 Gew.-%, ganz besonders bevorzugt 8 bis 12 Gew.-%, in einer Tätowiertinte eingesetzt werden. Die Menge an ein oder mehreren Lokalanästhetika in der Lösung kann je nach Bedarf variiert werden. Dies richtet sich nach jedem Einzelnen, der jeweiligen Schmerzempfindlichkeit, der zu tätowierenden Körperstelle und der Art und Größe des gewünschten Motivs.

Wenn das Lokalanästhetikum in der Tätowierzusammensetzung vorliegt, hat dies den Vorteil, dass nur eine Lösung zum Tätowieren eingesetzt werden muss. Die anästhesierend wirkende Tätowiertinte hat hierbei nicht nur die farbgebende Funktion für ein Tattoo, sondern wirkt gleichzeitig auch schmerzstillend in der Haut und verringert den Schmerz beim Tätowieren in gewünschtem Maße oder schaltet diesen nahezu gänzlich aus.

Wenn das Lokalanästhetikum in einer separaten Zusammensetzung zur Tätowierzusammensetzung eingesetzt wird, beispielsweise einer 4 Gew-%igen Lösung von Lidocainhydrochlorid in sterilem Wasser, kann diese unabhängig mit ein oder mehreren Tätowiertinten gleichzeitig zum Einsatz kommen. Hierdurch kann während des Tätowierens die Dosierung des oder der Lokalanästhetika in einfacher Weise geändert und entsprechend angepasst werden. So kann die Konzentration an Lokalanästhetikum während des Tätowierens in einfacher Weise variiert werden.

Beispielsweise kann mit einer flüssigen Zusammensetzung mit geringer Konzentration an Lokalanästhetikum begonnen werden. Sollte sich dann während des Tätowierens herausstellen, dass diese Konzentration nicht ausreichend ist und eine etwas höhere Konzentration erforderlich ist, kann bei einer separaten Bereitstellung der Lokalanästhetika-Lösung eine höher dosierte Lösung Verwendung finden. Dies kann je nach zu tätowierender Körperstelle und entsprechend der Schmerzempfindlichkeit auf jeden Einzelnen abgestimmt werden.

Das oder die Lokalanästhetika können in der flüssigen Zusammensetzung in entsprechender Menge bzw. Konzentration vorliegen, wie bereits beschrieben. Bei der 2. Alternative mit 2 Zusammensetzungen ist zu beachten, dass sich die Konzentration des oder der Lokalanästhetika durch zusätzliches Eintauchen der Nadel/n in die Tätowiertinte entsprechend verdünnt. Dies sollte bei der Dosierung von Lokalanästhetikum berücksichtigt werden.

Weiterhin wird auch die elektrische Tätowiermaschine in entsprechender Weise vorbereitet. Die Bedienung der elektrischen Tätowiermaschine erfolgt in der Regel über einen Fußschalter, wodurch diese ein- und ausgeschaltet wird. Das Tätowieren an sich wird jedoch nicht kontinuierlich durchgeführt, sondern es wird die Tätowiertinte und das Lokalanästhetikum durch Eintauchen der Nadel(n) in einen Behälter aufgenommen und in kurzen Intervallen am Stück tätowiert und immer wieder unterbrochen, um weitere Tätowiertinte und Lokalanästhetikum auf die Nadel(n) zu bekommen.

Beim Tätowieren können eine Vielzahl von Parametern ausgewählt und eingestellt werden: So können bei den verschiedenen am Markt erhältlichen Tätowiervorrichtungen (Colour Packer, Liner, Shader) bereits eine Vielzahl von Einstellungen, wie zum Beispiel die Eindringtiefe in die Haut, verändert werden. Ferner kann zwischen verschiedenen Arten von Nadeln, zum Beispiel anhand der verschiedenen Nadelstärken mit unterschiedlichen Schliffen der Nadelspitze (short taper, medium taper, long taper), die je nach Konsistenz der zu verwendenden Tätowiertinte, der Art des aufzubringenden Tattoos und der Linienführung (Nadeln in Form von round liner, round shader, flat magnum) unterschiedliche Ergebnisse liefern, ausgewählt werden. Auch hinsichtlich der Farben der einzelnen Tätowiertinten gibt es zahlreiche Unterschiede und eine Fülle an Herstellern, so dass hier eine entsprechende Erfahrung bei der Auswahl nützlich ist. Es ist daher empfehlenswert, wenn das Tätowieren von einem erfahrenen Tätowierer vorgenommen wird.

Neben der Vorbereitung der Materialien und hygienischen Anforderungen wird auch die Haut des zu Tätowierenden vorbereitet. Als Vorbereitung auf das Tätowieren wird die Körperstelle, an der das Tattoo gestochen werden soll, zunächst gesäubert und desinfiziert. Falls nötig, kann die Haut rasiert werden. Dann wird das ausgewählte Motiv freihändig oder mittels einer Matrize auf die Haut übertragen.

Die Tätowiervorrichtung wird dann angeschaltet, die ein oder mehreren Nadeln werden in eine flüssige Tätowiertinte, enthaltend ein oder mehrere Lokalanästhetika, getaucht, die sich in einem entsprechenden Behälter befindet. Alternativ werden die ein oder mehreren Nadeln bevorzugt zunächst in eine anästhesierende Zusammensetzung und dann in die flüssige Tätowiertinte getaucht, die sich jeweils in unterschiedlichen Behältern befinden.

Mit Hilfe der Tätowiermaschine sticht der Tätowierer dann die Umrisse auf die Haut und säubert diese, die Haut wird dabei mit der einen Hand unter Spannung gehalten, während die andere Hand das Bild eintätowiert. Blut und überschüssige Tattoo-Farbe werden mit einem Einwegtuch abgewischt.

Da es beim Tätowieren bluten kann, ist es zweckmäßig, wenn Einweg-Handschuhe und Einweg-Nadeln verwendet werden, um ein mögliches Infektionsrisiko des Tätowierers und des zu Tätowierenden auszuschließen. Je nach Art des Motivs werden Linien, Schattierungen oder ganze Flächen tätowiert.

Bei jedem Absetzen während des Tätowierens wird immer wieder - je nachdem, welche Variante gewählt wurde - in einen oder beide Behälter eingetaucht, um gleichzeitig Tinte und Lokalanästhetikum auf der Nadel vorliegen zu haben.

Zuletzt wird der tätowierte Bereich erneut gesäubert und ein Verband angelegt.

Im vorliegenden Fall handelt es sich um ein klassisches Tätowieren, das nicht für den Einsatz in speziellen kosmetischen Verfahren, wie beispielsweise zum Auffüllen von Hautfalten, ähnlicher Hautphänomene oder Verändern der Haut oder Hautbeschaffenheit an sich, gedacht ist und soll in der vorliegenden Erfindung auch nicht hierfür verwendet werden. Es sollen auch keine Hautentfernungen oder andere Veränderungen der Haut, wie beispielsweise Farbveränderungen durch Abgabe von Wirkstoffen oder dergleichen durchgeführt werden, sondern ausschließlich das klassische Tätowieren unter Auftragen eines Motivs auf die 2. Hautschicht erfolgen.

Tätowieren soll im Gegensatz zu einer Injektion verstanden werden, wobei Substanzen unter die Haut (subkutan) eingebracht werden, wohingegen beim Tätowieren Färbemittel in die Haut eingebracht wird.

Ein Bodypainting wird hier nicht als Tätowierung verstanden, da dieses nicht permanent vorliegt.

Es sei darauf hingewiesen, dass das Tätowieren an sich zu der Kategorie von Verfahren gehört, die nur zur Verschönerung des menschlichen oder tierischen Körpers dienen, wobei das Tätowieren auch gewerbliche Anwendung in Unternehmen findet, wie Tattoo-Studios, Kosmetikstudios oder Schönheitssalons. Das Tätowieren gehört zu den kosmetischen Verfahren, die allein zu ästhetischen Zwecken dienen. Ein therapeutisches Verfahren liegt hier nicht vor, da ein verbessertes ästhetisches Erscheinungsbild der behandelten Person bzw. eine Markierung eines Tiers bezweckt und keine Krankheit behandelt wird.

Nachfolgend sollen einige Ausführungsbeispiele für Tätowiertinten angegeben werden, welche die vorliegende Erfindung jedoch nicht hierauf beschränken sollen.

### Erfindungsgemäße Ausführungsbeispiele für Anästhetikum und Hilfsstoffe

In den nachfolgenden Ausführungsbeispielen wurde als Lokalanästhetikum Lidocainhydrochlorid 4 Gew.-% (40 mg/ ml) in wässeriger Lösung (Wasser für Injektionszwecke) eingesetzt.

Die verwendeten Hilfsstoffe waren:
- Wasser für Injektionszwecke,
- Ethanol 96 % (V/V),
- Natronlauge zur Einstellung des pH-Wertes und
- Poloxamer.

### Beispiel 1

Die flüssige Zusammensetzung einer schwarzen Tätowiertinte wurde verwendet. Die Tätowiertinte enthielt die zuvor angegebenen Hilfsstoffe.

Als Lokalanästhetikum wurde Lidocainhydrochlorid 4 Gew.-% (40 mg/ml) in wässeriger Lösung verwendet.

Das Lokalanästhetikum in wässeriger Lösung in einer Konzentration von 4 Gew.-% wurde zur Tätowiertinte zugegeben und gut vermischt.

Die Tätowiertinte zeigte eine sehr gute Farb- und Lichtechtheit. Mit der anästhetisierend wirkenden Tätowiertinte konnte praktisch schmerzfrei tätowiert werden.

### Beispiel 2

Die flüssige Zusammensetzung einer schwarzen Tätowiertinte wurde verwendet. Die Tätowiertinte enthielt die zuvor angegebenen Hilfsstoffe.

Als Lokalanästhetikum wurde Lidocainhydrochlorid in einer Konzentration von 4 Gew.-% (40 mg/ml) in einer separaten Lösung in sterilem Wasser, geeignet zur Injektion, verwendet.

Beim Tätowieren wurden die Nadeln zunächst in die Lidocainhydrochlorid-Lösung und unmittelbar anschließend in die Tätowiertinte getaucht. Dann wurde tätowiert. Bei jeder Unterbrechung des Tätowierens wurden die Nadeln wieder nacheinander in beide Lösungen getaucht.

Die Tätowiertinte zeigte auch in diesem Beispiel eine sehr gute Farb- und Lichtechtheit.

Auch unter Verwendung der beiden Lösungen in Kombination konnte praktisch schmerzfrei tätowiert werden.

## Patentansprüche

1. Kombination zum Tätowieren, umfassend eine Tätowiertinte und ein oder mehrere Lokalanästhetika für die Haut, wobei
die Tätowiertinte und die ein oder mehreren Lokalanästhetika zusammen in einer flüssigen Zusammensetzung vorliegen oder
die Tätowiertinte in einer ersten flüssigen Zusammensetzung und die ein oder mehreren Lokalanästhetika in einer zweiten flüssigen Zusammensetzung vorliegen, wobei ein Anteil der ersten Zusammensetzung und ein Anteil der zweiten Zusammensetzung vor und während dem Tätowieren zusammen in einer flüssigen Zusammensetzung vorliegen,
**dadurch gekennzeichnet, dass**
die flüssige Zusammensetzung entzündungshemmende Mittel enthält und
das entzündungshemmende Mittel ausgewählt ist aus Vitamin F, Vitamin E, ungesättigten Fettsäuren, Rutin, Bioflavanoiden, Sanddorn-Öl, Olivenöl, Jojobaöl, ätherischem Kamillenöl, Betamethason, Dexamethason und Mischungen dieser.

2. Kombination nach Anspruch 1,
**dadurch gekennzeichnet, dass**
die flüssige Zusammensetzung, umfassend die Tätowiertinte und ein oder mehrere Lokalanästhetika, eine Zusammensetzung auf Wasser-Basis darstellt oder die erste und/oder zweite Zusammensetzung jeweils Zusammensetzungen auf Wasser-Basis darstellen.

3. Kombination nach mindestens einem der vorangehenden Ansprüche,
**dadurch gekennzeichnet, dass** ein Anteil der ersten Zusammensetzung und ein Anteil der zweiten Zusammensetzung erst auf der oder den Nadeln einer Tätowiervorrichtung zusammen vorliegen.

4. Kombination nach mindestens einem der vorangehenden Ansprüche 1 bis 3,
**dadurch gekennzeichnet, dass**
das oder die Lokalanästhetika ausgewählt sind aus der Gruppe, bestehend aus Lidocain, Mepivacain, Prilocain, Articain, Bupivacain, Dibucain, Ropivacain, Etidocain, Dyclonin, Procain, Benzocain, 2-Chlorprocain, Oxybuprocain, Tetracain, Fomocain, Pramocain,
Kampfer, Lignocain, Etidocain, Levobupivacain, Oxyprocain, Hexylcain, Dibucain, Piperocaine, Butamben, Butambenpikrate, Dimethisoquinhydrochlorid, Diperodon, Dyclonin, Ketamin, p-Buthylaminobenzoesäure und Pramoxin und pharmazeutisch akzeptable Salze sowie Mischungen dieser, bevorzugt Lidocain und Benzocain, deren pharmazeutisch akzeptable Salze sowie Mischungen dieser,
wobei
das oder die Lokalanästhetika in der flüssigen Zusammensetzung oder in der zweiten flüssigen Zusammensetzung bevorzugt in einer Konzentration im Bereich von 4 bis 20 Gew.-%, noch bevorzugter 6 bis 15 Gew.-%, ganz besonders bevorzugt 8 bis 12 Gew.-%, vorliegen.

5. Kombination nach mindestens einem der vorangehenden Ansprüche 1 bis 4, **dadurch gekennzeichnet, dass**
das oder die Lokalanästhetika in der flüssigen Zusammensetzung oder in der zweiten flüssigen Zusammensetzung je nach dem Schmerzempfinden, der zu tätowierenden Körperstelle und dem Motiv in entsprechender Konzentration vorliegen,
wobei die Konzentration bevorzugt im folgenden Bereich liegt:
niedrige Konzentration im Bereich von 4 bis 7,9 Gew.-%,
mittlere Konzentration im Bereich von 8 bis 12 Gew.-% oder
hohe Konzentration im Bereich von 12,1 bis 20 Gew.-%.

6. Kombination nach mindestens einem der vorangehenden Ansprüche 1 bis 5,
**dadurch gekennzeichnet, dass**
die Tätowiertinte in der flüssigen Zusammensetzung oder der ersten flüssigen Zusammensetzung die folgenden Bestandteile umfasst:
ein oder mehrere physiologisch akzeptable Färbemittel, die ausgewählt sind aus der Gruppe, bestehend aus organischen oder anorganischen Pigmenten, natürlichen oder synthetischen Farbstoffen und Kombinationen hiervon;
ein oder mehrere physiologisch akzeptable Lösungsmittel, die ausgewählt sind aus der Gruppe, bestehend aus Alkoholen und Wasser;
und optional ein oder mehrere physiologisch akzeptable Hilfsstoffe, die ausgewählt sind aus der Gruppe, bestehend aus Lösungsvermittlern, Dispergiermitteln, Bindemitteln,
Verdickungsmitteln, Viskositätsregulatoren, Konservierungsmitteln, Stabilisatoren, Emulgatoren, Antioxidationsmitteln, Duftstoffen, Puffern, Mitteln zur Regulierung des pH-Werts, Befeuchtungs- oder Feuchthaltemitteln, Antiseptika und Kombinationen dieser,
wobei in der flüssigen Zusammensetzung zusätzlich ein oder mehrere Lokalanästhetika
und in der flüssigen Zusammensetzung und der ersten flüssigen Zusammensetzung ein entzündungshemmendes Mittel, ausgewählt aus Vitamin F, Vitamin E, ungesättigten Fettsäuren, Rutin, Bioflavanoiden, Sanddorn-Öl, Olivenöl, Jojobaöl, ätherischem Kamillenöl, Betamethason, Dexamethason und Mischungen dieser,
enthalten sind.

7. Kombination nach mindestens einem der vorangehenden Ansprüche 1 bis 6,
**dadurch gekennzeichnet, dass**
die flüssige Zusammensetzung ein oder mehrere Lokalanästhetika und entzündungshemmende Mittel enthält, die ausgewählt sind aus Vitamin F, Vitamin E, ungesättigten Fettsäuren, Rutin, Bioflavanoiden, Sanddorn-Öl, Olivenöl, Jojobaöl, ätherischem Kamillenöl, Betamethason, Dexamethason und Mischungen dieser und die folgenden Bestandteile umfasst: 30 bis 95 Gew% ein oder mehrere Lösungsmittel, bevorzugt Wasser;
1 bis 35 Gew% ein oder mehrere Färbemittel;
0 bis 15 Gew% ein oder mehrere Hilfsstoffe; und
4 bis 20 Gew% ein oder mehrere Lokalanästhetika.

8. Kombination zum Tätowieren nach einem der vorangehenden Ansprüche 1 bis 7 zur Schmerzlinderung oder -vermeidung während des Tätowierens.

9. Kombination nach Anspruch 8
**dadurch gekennzeichnet, dass**
die Tätowiertinte und die ein oder mehreren Lokalanästhetika zusammen in einer flüssigen Zusammensetzung eingesetzt werden und vor und während dem Tätowieren ein oder mehrere
Nadeln einer Tätowiervorrichtung in die flüssige Zusammensetzung eingetaucht werden;
oder
die Tätowiertinte in einer ersten flüssigen Zusammensetzung und die ein oder mehreren Lokalanästhetika in einer zweiten flüssigen Zusammensetzung eingesetzt werden und vor und während dem Tätowieren ein oder mehrere Nadeln einer Tätowiervorrichtung
zunächst in die erste und dann in die zweite flüssige Zusammensetzung
oder
zunächst in die zweite und dann in die erste flüssige Zusammensetzung eingetaucht werden,
bevor der jeweilige Tätowiervorgang durchgeführt wird.

10. Kombination nach Anspruch 9,
**dadurch gekennzeichnet, dass**
das Eintauchen der ein oder mehreren Nadeln in die flüssige Zusammensetzung oder die erste und zweite flüssige Zusammensetzung oder die zweite und erste flüssige Zusammensetzung während des Tätowierens wiederholt wird, bevorzugt bei jeder Unterbrechung des Tätowiervorgangs durchgeführt wird, bis das Tätowieren beendet ist.

11. Kombination nach mindestens einem der vorangehenden Ansprüche 8, 9 oder 10,
**dadurch gekennzeichnet, dass**
als flüssige Zusammensetzung, umfassend die Tätowiertinte und ein oder mehrere Lokalanästhetika, eine Zusammensetzung auf Wasser-Basis eingesetzt wird oder als die erste und/oder zweite Zusammensetzung jeweils eine Zusammensetzung auf Wasser-Basis eingesetzt wird.

12. Kombination nach mindestens einem der vorangehenden Ansprüche 8 bis 11,
**dadurch gekennzeichnet, dass**
das Lokalanästhetikum ausgewählt wird aus der Gruppe, bestehend aus Lidocain, Mepivacain, Prilocain, Articain, Bupivacain, Dibucain, Ropivacain, Etidocain, Dyclonin, Procain, Benzocain, 2-Chlorprocain, Oxybuprocain, Tetracain, Fomocain, Pramocain,
Kampfer, Lignocain, Etidocain, Levobupivacain, Oxyprocain, Hexylcain, Dibucain, Piperocaine, Butamben, Butambenpikrate, Dimethisoquinhydrochlorid, Diperodon, Dyclonin, Ketamin, p-Buthylaminobenzoesäure und Pramoxin und pharmazeutisch akzeptable Salze sowie Mischungen dieser, bevorzugt Lidocain und Benzocain, deren pharmazeutisch akzeptable Salze sowie Mischungen dieser,
wobei
das oder die Lokalanästhetika in einer Konzentration im Bereich von 4 bis 20 Gew.-%, noch bevorzugter 6 bis 15 Gew.-%, ganz besonders bevorzugt 8 bis 12 Gew.-%, eingesetzt werden.

13. Kombination nach mindestens einem der vorangehenden Ansprüche 8 bis 12,
**dadurch gekennzeichnet, dass**
die Konzentration an ein oder mehreren Lokalanästhetika in der flüssigen Zusammensetzung oder in der zweiten flüssigen Zusammensetzung variiert wird, wobei die Konzentration bevorzugt aus den folgenden Bereichen ausgewählt wird:
niedrige Konzentration im Bereich von 4 bis 7,9 Gew.-%,
mittlere Konzentration im Bereich von 8 bis 12 Gew.-% oder
hohe Konzentration im Bereich von 12,1 bis 20 Gew.-%.

14. Kombination nach mindestens einem der vorangehenden Ansprüche 8 bis 13,
**dadurch gekennzeichnet, dass**
Tätowiertinte in einer flüssigen Zusammensetzung oder einer ersten flüssigen Zusammensetzung, umfassend ein oder mehrere Lokalanästhetika für die Haut und entzündungshemmende Mittel, ausgewählt aus Vitamin F, Vitamin E, ungesättigten Fettsäuren, Rutin, Bioflavanoiden, Sanddorn-Öl, Olivenöl, Jojobaöl, ätherischem Kamillenöl, Betamethason, Dexamethason und Mischungen dieser, mit den folgenden Bestandteilen eingesetzt wird:
ein oder mehrere physiologisch akzeptable Färbemittel, die ausgewählt werden aus der Gruppe, bestehend aus organischen oder anorganischen Pigmenten, natürlichen oder synthetischen Farbstoffen und Kombinationen hiervon;
ein oder mehrere physiologisch akzeptable Lösungsmittel, die ausgewählt werden aus der Gruppe, bestehend aus Alkoholen und Wasser;
und optional ein oder mehrere
physiologisch akzeptable Hilfsstoffe, die ausgewählt werden aus der Gruppe, bestehend aus Lösungsvermittlern, Dispergiermitteln, Bindemitteln, Verdickungsmitteln, Viskositätsregulatoren, Konservierungsmitteln, Stabilisatoren, Emulgatoren, Antioxidationsmitteln, Duftstoffen, Puffern, Mitteln zur Regulierung des pH-Werts, Befeuchtungs- oder Feuchthaltemitteln, Antiseptika und Kombinationen dieser.

15. Kombination nach mindestens einem der vorangehenden Ansprüche 8 bis 14,
**dadurch gekennzeichnet, dass**
die flüssige Zusammensetzung, umfassend ein oder mehrere Lokalanästhetika und entzündungshemmende Mittel, die ausgewählt sind aus Vitamin F, Vitamin E, ungesättigten Fettsäuren, Rutin, Bioflavanoiden, Sanddorn-Öl, Olivenöl, Jojobaöl, ätherischem Kamillenöl, Betamethason, Dexamethason und Mischungen dieser, mit den folgenden Bestandteilen eingesetzt wird:
30 bis 95 Gew% ein oder mehrere Lösungsmittel, bevorzugt Wasser;
1 bis 35 Gew% ein oder mehrere Färbemittel;
0 bis 15 Gew% ein oder mehrere Hilfsstoffe; und
4 bis 20 Gew% ein oder mehrere Lokalanästhetika.

## Claims

1. A combination for tattooing comprising a tattoo ink and one or several local anesthetics for the skin, wherein
the tattoo ink and the one or several local anesthetics are present together in a liquid composition, or
the tattoo ink is present in a first liquid composition and the one or several local anesthetics are present in a second liquid composition, wherein a portion of the first composition and a portion of the second composition are present together in a liquid composition prior to and during tattooing,
**characterized in that**
the liquid composition contains anti-inflammatory agents, and
the anti-inflammatory agent is selected from vitamin F, vitamin E, unsaturated fatty acids, rutin, bioflavanoids, sea buckthorn oil, olive oil, jojoba oil, chamomile essential oil, betamethasone, dexamethasone and mixtures thereof.

2. Combination according to claim 1,
**characterized in that**
the liquid composition comprising the tattoo ink and one or several local anesthetics is a water-based composition or the first and/or second composition are each water-based compositions.

3. Combination according to at least one of the preceding claims,
**characterized in that** a portion of the first composition and a portion of the second composition are only present together on the needle or needles of a tattooing device.

4. Combination according to at least one of the preceding claims 1 to 3,
**characterized in that**
the local anesthetic(s) is or are selected from the group consisting of lidocaine, mepivacaine, prilocaine, articaine, bupivacaine, dibucaine, ropivacaine, etidocaine, dyclonine, procaine, benzocaine, 2-chloroprocaine, oxybuprocaine, tetracaine, fomocaine, pramocaine, camphor, lignocaine, etidocaine, levobupivacaine, oxyprocaine, hexylcaine, dibucaine, piperocaine, butambene, butambene picrate, dimethisoquine hydrochloride, diperodon, dyclonine, ketamine, p-butylaminobenzoic acid and pramoxine and pharmaceutically acceptable salts and mixtures thereof, preferably lidocaine and benzocaine, their pharmaceutically acceptable salts and mixtures thereof,
wherein
the local anesthetic(s) is or are present in the liquid composition or the second liquid composition preferably in a concentration ranging from 4 to 20% by weight, more preferably 6 to 15% by weight, very particularly preferably 8 to 12% by weight.

5. Combination according to at least one of the preceding claims 1 to 4, **characterized in that**
the local anesthetic(s) in the liquid composition or in the second liquid composition are present in the respective concentration depending on the pain sensation, the part of the body to be tattooed and the motif,
wherein the concentration is preferably in the following range:
low concentration in the range of 4 to 7.9% by weight,
medium concentration in the range of 8 to 12% by weight or
high concentration in the range of 12.1 to 20% by weight.

6. Combination according to at least one of the preceding claims 1 to 5,
**characterized in that**
the tattoo ink in the liquid composition or the first liquid composition comprises the following components:
one or several physiologically acceptable colorants selected from the group consisting of organic or inorganic pigments, natural or synthetic dyes and combinations thereof;
one or several physiologically acceptable solvents selected from the group consisting of alcohols and water;
and optionally one or several physiologically acceptable excipients selected from the group consisting of solubilizers, dispersants, binders, thickeners, viscosity regulators, preservatives, stabilizers, emulsifiers, antioxidants, fragrances, buffers, pH regulators, humectants or wetting agents, antiseptics and combinations thereof,
wherein the liquid composition additionally contains one or several local anesthetics
and the liquid composition and the first liquid composition contains an anti-inflammatory agent selected from vitamin F, vitamin E, unsaturated fatty acids, rutin, bioflavanoids, sea buckthorn oil, olive oil, jojoba oil, chamomile essential oil, betamethasone, dexamethasone and mixtures thereof.

7. Combination according to at least one of the preceding claims 1 to 6,
**characterized in that**
the liquid composition comprises one or several local anesthetics and anti-inflammatory agents selected from vitamin F, vitamin E, unsaturated fatty acids, rutin, bioflavanoids, sea buckthorn oil, olive oil, jojoba oil, chamomile essential oil, betamethasone, dexamethasone and mixtures thereof and the following components:
30 to 95% by weight of one or several solvents, preferably water;
1 to 35% by weight of one or several colorants;
0 to 15% by weight of one or several excipients; and
4 to 20% by weight of one or several local anesthetics.

8. Combination for tattooing according to any one of the preceding claims 1 to 7 for reducing or avoiding pain during tattooing.

9. Combination according to claim 8,
**characterized in that**
the tattoo ink and the one or several local anesthetics are used together in a liquid composition and, before and during tattooing, one or several needles of a tattooing device are immersed in the liquid composition;
or
the tattoo ink is used in a first liquid composition and the one or several local anesthetics are used in a second liquid composition and, before and during tattooing, one or several needles of a tattooing device are immersed first in the first and then in the second liquid composition,
or
first in the second and then in the first liquid composition
prior to performing the respective tattooing operation.

10. Combination according to claim 9,
**characterized in that**
the immersion of the one or several needles in the liquid composition or the first and second liquid composition or the second and first liquid composition is repeated during tattooing, preferably is carried out at each interruption of the tattooing process until tattooing is finished.

11. Combination according to at least one of the preceding claims 8, 9 or 10,
**characterized in that**
a water-based composition is used as the liquid composition comprising the tattoo ink and one or several local anesthetics, or a water-based composition is used as the first and/or second composition respectively.

12. Combination according to at least one of the preceding claims 8 to 11,
**characterized in that**
the local anesthetic is selected from the group consisting of lidocaine, mepivacaine, prilocaine, articaine, bupivacaine, dibucaine, ropivacaine, etidocaine, dyclonine, procaine, benzocaine, 2-chloroprocaine , oxybuprocaine, tetracaine, fomocaine, pramocine, camphor, lignocaine, etidocaine, levobupivacaine, oxyprocaine, hexylcaine, dibucaine, piperocaine, butambene, butambene picrate, dimethisoquine hydrochloride, diperodon, dyclonine, ketamine, p-butylaminobenzoic acid and pramoxine and pharmaceutically acceptable salts as well as mixtures thereof, preferably lidocaine and benzocaine, their pharmaceutically acceptable salts as well as mixtures thereof, wherein
the local anesthetic(s) is or are used in a concentration in the range from 4 to 20% by weight, more preferably 6 to 15% by weight, very particularly preferably 8 to 12% by weight.

13. Combination according to at least one of the preceding claims 8 to 12,
**characterized in that**
the concentration of one or several local anesthetics in the liquid composition or in the second liquid composition is varied, wherein the concentration is preferably selected from the following ranges:
low concentration in the range from 4 to 7.9 % by weight,
medium concentration in the range of 8 to 12% by weight or
high concentration in the range of 12.1 to 20% by weight.

14. Combination according to at least one of the preceding claims 8 to 13,
**characterized in that**
tattoo ink is used in a liquid composition or a first liquid composition comprising one or several local anesthetics for the skin and anti-inflammatory agents selected from vitamin F, vitamin E, unsaturated fatty acids, rutin, bioflavanoids, sea buckthorn oil, olive oil, jojoba oil, chamomile essential oil, betamethasone, dexamethasone and mixtures thereof, with the following components:
one or several physiologically acceptable colorants selected from the group consisting of organic or inorganic pigments, natural or synthetic dyes and combinations thereof;
one or several physiologically acceptable solvents selected from the group consisting of alcohols and water;
and optionally one or several physiologically acceptable excipients selected from the group consisting of solubilizers, dispersants, binders, thickeners, viscosity regulators, preservatives, stabilizers, emulsifiers, antioxidants, fragrances, buffers, pH regulators, humectants or wetting agents, antiseptics and combinations thereof.

15. Combination according to at least one of the preceding claims 8 to 14,
**characterized in that**
the liquid composition comprising one or several local anesthetics and anti-inflammatory agents selected from vitamin F, vitamin E, unsaturated fatty acids, rutin, bioflavanoids, sea buckthorn oil, olive oil, jojoba oil, chamomile essential oil, betamethasone, dexamethasone and mixtures thereof, is used with the following components:
30 to 95% by weight of one or several solvents, preferably water;
1 to 35% by weight of one or several colorants;
0 to 15% by weight of one or several excipients; and
4 to 20% by weight of one or several local anesthetics.

## Revendications

1. Association, destinée au tatouage, comprenant une encre à tatouer et un ou plusieurs anesthésiants locaux pour la peau,
l'encre à tatouer et l'un ou les plusieurs anesthésiants locaux se présentent conjointement dans une composition liquide ou
l'encre à tatouer se présentant dans une première composition liquide et l'un ou les plusieurs anesthésiants locaux se présentant dans une deuxième composition liquide, avant et pendant l'opération de tatouage, une part de la première composition et une part de la deuxième composition se présentant conjointement dans une composition liquide,
**caractérisée en ce que**
la composition liquide contient des agents anti-inflammatoires et
l'agent anti-inflammatoire est sélectionné parmi la vitamine F, la vitamine E, les acides gras insaturés, la rutine, les bio-flavonoïdes, l'huile d'argousier, l'huile d'olive, l'huile de jojoba, l'huile essentielle de camomille, la bétaméthasone, la dexaméthasone et leurs mélanges.

2. Association selon la revendication 1,
**caractérisée en ce que**
la composition liquide, comprenant l'encre à tatouer et un ou plusieurs anesthésiants locaux constitue une composition sur base aqueuse ou la première et/ou la deuxième composition constituent chacune des compositions sur base aqueuse.

3. Association selon au moins l'une quelconque des revendications précédentes,
**caractérisée en ce qu'**une part de la première composition et une part de la deuxième composition ne se présentent conjointement qu'une fois sur l'aiguille ou sur les aiguilles d'un dispositif de tatouage.

4. Association selon au moins l'une quelconque des revendications précédentes 1 à 3, **caractérisée en ce que**
le ou les anesthésiants locaux sont sélectionnés dans le groupe comprenant la lidocaïne, la mépivacaïne, la prilocaïne, l'articaïne, la bupivacaïne, la dibucaïne, la ropivacaïne, l'étidocaïne, la dyclonine, la procaïne, l'enzocaïne, la 2-chloroprocaïne, l'oxybuprocaïne, la tétracaïne, la fomocaïne, la pramocaïne, le camphre, la lignocaïne, l'étidocaïne, la lévobupivacaïne, l'oxyprocaïne, l'hexylcaïne, la dibucaïne, les piperocaïnes, le butambène, les picrates de butambène, l'hydrochlorure de diméthisoquine, le dipérodon, la dyclonine, la kétamine, l'acide p-buthylaminobenzoïque et la pramoxine ainsi que des sels pharmaceutiquement acceptables et leurs mélanges, de préférence la lidocaïne et la benzocaïne, leurs sels pharmaceutiquement acceptables et leurs mélanges,
le ou les anesthésiants locaux se présentant dans la composition liquide ou dans la deuxième composition liquide, de préférence dans une concentration de l'ordre de 4 à 20 % en poids, de manière plus préférentielle, de 6 à 15 % en poids, de manière tout particulièrement préférentielle, de 8 à 12 % en poids.

5. Association selon au moins l'une quelconque des revendications précédentes 1 à 4, **caractérisée en ce que**
le ou les anesthésiants locaux se présentent dans la composition liquide ou dans la deuxième composition liquide dans une concentration correspondante, en fonction de la sensation de douleur ressentie dans la partie du corps qui doit être tatouée et du motif,
la concentration étant de préférence de l'ordre suivant :
une basse concentration de l'ordre de 4 à 7,9 % en poids,
une concentration moyenne de l'ordre de 8 à 12 % en poids ou
une concentration élevée de l'ordre de 12,1 à 20 % en poids.

6. Association selon au moins l'une quelconque des revendications précédentes 1 à 5, **caractérisée en ce que**
l'encre à tatouer dans la composition liquide ou dans la première composition liquide comprend les constituants suivants :
un ou plusieurs colorants physiologiquement acceptables qui sont sélectionnés dans le groupe comprenant les pigments organiques ou inorganiques, les colorants naturels ou synthétiques et leurs associations ;
un ou plusieurs solvants physiologiquement acceptables, qui sont sélectionnés dans le groupe comprenant les alcools et l'eau ;
et en option, un ou plusieurs excipients physiologiquement acceptables qui sont sélectionnés dans le groupe comprenant les agents solubilisants, les agents dispersants, les liants, les épaississants, les régulateurs de viscosité, les conservateurs, les stabilisateurs, les émulsifiants, les antioxydants, les parfums, les tampons, les agents régulateurs de pH, les humectants ou les rétenteurs d'humidité, les antiseptiques et leurs associations,
dans la composition liquide étant contenus additionnellement un ou plusieurs anesthésiants locaux
et dans la composition liquide ainsi que dans la première composition liquide étant contenu un agent anti-inflammatoire, sélectionné parmi la vitamine F, la vitamine E, les acides gras insaturés, la rutine, les bio-flavonoïdes, l'huile d'argousier, l'huile d'olive, l'huile de jojoba, l'huile essentielle de camomille, la bétaméthasone, la dexaméthasone et leurs mélanges.

7. Association selon au moins l'une quelconque des revendications précédentes 1 à 6, **caractérisée en ce que**
la composition liquide contient un ou plusieurs anesthésiants locaux et des agents anti-inflammatoires qui sont sélectionnés parmi la vitamine F, la vitamine E, les acides gras insaturés, la rutine, les bio-flavonoïdes, l'huile d'argousier, l'huile d'olive, l'huile de jojoba, l'huile essentielle de camomille, la bétaméthasone, la dexaméthasone et leurs mélanges et comprend les constituants suivants :
de 30 à 95 % en poids d'un ou de plusieurs solvants, de préférence de l'eau ;
de 1 à 35 % en poids d'un ou plusieurs colorants ;
de 0 à 15 % en poids d'un ou plusieurs excipients ; et
de 4 à 20 % en poids d'un ou de plusieurs anesthésiants locaux.

8. Association, destinée au tatouage selon l'une quelconque des revendications précédentes 1 à 7, à vocation d'atténuer ou d'éviter la douleur pendant l'opération de tatouage.

9. Association selon la revendication 8 **caractérisée en ce que**
l'on met en œuvre conjointement l'encre à tatouer et l'un ou les plusieurs anesthésiants locaux dans une composition liquide et qu'avant ou pendant l'opération de tatouage, l'on trempe une ou plusieurs aiguilles d'un dispositif de tatouage dans la composition liquide ;
ou
**en ce que** l'on met en œuvre l'encre à tatouer dans une première composition liquide et l'un ou les plusieurs anesthésiants locaux dans une deuxième composition liquide et qu'avant ou pendant l'opération de tatouage, l'on trempe une ou plusieurs aiguilles d'un dispositif de tatouage d'abord dans la première et ensuite dans la deuxième composition liquide
ou
d'abord dans la deuxième et ensuite dans la première composition liquide,
avant de procéder à l'opération de tatouage en question.

10. Association selon la revendication 9,
**caractérisée en ce que**
l'on répète pendant l'opération de tatouage l'action consistant à tremper l'une ou les plusieurs aiguilles dans la composition liquide ou dans la première et la deuxième composition liquide ou dans la deuxième et la première composition liquide, **en ce qu'**on la réalise de préférence à chaque interruption de l'opération de tatouage, jusqu'à son achèvement.

11. Association selon au moins l'une quelconque des revendications précédentes 8, 9 ou 10,
**caractérisée en ce que**
l'on met en œuvre en tant que composition liquide, comprenant l'encre à tatouer et un ou plusieurs anesthésiants locaux une composition sur base aqueuse ou **en ce que** l'on met en œuvre en tant que la première et/ou la deuxième composition chaque fois une composition sur base aqueuse.

12. Association selon au moins l'une quelconque des revendications précédentes 8 à 11,
**caractérisée en ce que**
l'anesthésiant local est sélectionné dans le groupe comprenant la lidocaïne, la mépivacaïne, la prilocaïne, l'articaïne, la bupivacaïne, la dibucaïne, la ropivacaïne, l'étidocaïne, la dyclonine, la procaïne, l'enzocaïne, la 2-chloroprocaïne, l'oxybuprocaïne, la tétracaïne, la fomocaïne, la pramocaïne, le camphre, la lignocaïne, l'étidocaïne, la lévobupivacaïne, l'oxyprocaïne, l'hexylcaïne, la dibucaïne, les piperocaïnes, le butambène, les picrates de butambène, l'hydrochlorure de diméthisoquine, le dipérodon, la dyclonine, la kétamine, l'acide p-buthylaminobenzoïque et la pramoxine ainsi que des sels pharmaceutiquement acceptables et leurs mélanges, de préférence la lidocaïne et la benzocaïne, leurs sels pharmaceutiquement acceptables et leurs mélanges,
l'on met en œuvre le ou les anesthésiants locaux dans une concentration de l'ordre de 4 à 20 % en poids, de manière encore plus préférentielle, de 6 à 15 % en poids, de manière tout particulièrement préférentielle, de 8 à 12 % en poids.

13. Association selon au moins l'une quelconque des revendications précédentes 8 à 12,
**caractérisée en ce que**
l'on fait varier la composition de l'un ou de plusieurs anesthésiants locaux dans la composition liquide ou dans la deuxième composition liquide, la concentration étant sélectionnée de préférence dans les ordres suivants :
une basse concentration de l'ordre de 4 à 7,9 % en poids,
une concentration moyenne de l'ordre de 8 à 12 % en poids ou
une concentration élevée de l'ordre de 12,1 à 20 % en poids.

14. Association selon au moins l'une quelconque des revendications précédentes 8 à 13,
**caractérisée en ce que**
l'on met en œuvre de l'encre à tatouer dans une composition liquide ou dans une première composition liquide, comprenant un ou plusieurs anesthésiants locaux pour la peau et agents anti-inflammatoires sélectionnés parmi la vitamine F, la vitamine E, les acides gras insaturés, la rutine, les bio-flavonoïdes, l'huile d'argousier, l'huile d'olive, l'huile de jojoba, l'huile essentielle de camomille, la bétaméthasone, la dexaméthasone et leurs mélanges, avec les constituants suivants :
un ou plusieurs colorants physiologiquement acceptables qui sont sélectionnés dans le groupe comprenant les pigments organiques ou inorganiques, les colorants naturels ou synthétiques et leurs associations ;
un ou plusieurs solvants physiologiquement acceptables, qui sont sélectionnés dans le groupe comprenant les alcools et l'eau ;
et en option, un ou plusieurs excipients physiologiquement acceptables qui sont sélectionnés dans le groupe comprenant les agents solubilisants, les agents dispersants, les liants, les épaississants, les régulateurs de viscosité, les conservateurs, les stabilisateurs, les émulsifiants, les antioxydants, les parfums, les tampons, les agents régulateurs de pH, les humectants ou les rétenteurs d'humidité, les antiseptiques et leurs associations.

15. Association selon au moins l'une quelconque des revendications précédentes 8 à 14,
**caractérisée en ce que**
l'on met en œuvre la composition liquide, comprenant un ou plusieurs anesthésiants locaux et des agents anti-inflammatoires, sélectionnés parmi la vitamine F, la vitamine E, les acides gras insaturés, la rutine, les bio-flavonoïdes, l'huile d'argousier, l'huile d'olive, l'huile de jojoba, l'huile essentielle de camomille, la bétaméthasone, la dexaméthasone et leurs mélanges, avec les constituants suivants :
de 30 à 95 % en poids d'un ou de plusieurs solvants, de préférence de l'eau ;
de 1 à 35 % en poids d'un ou plusieurs colorants ;
de 0 à 15 % en poids d'un ou plusieurs excipients ; et
de 4 à 20 % en poids d'un ou de plusieurs anesthésiants locaux.
